(19) Europäisches Patentamt
European Patent Office
Office européen des brevets

(11) Veröffentlichungsnummer: **0 350 742**
**A1**

# (12) EUROPÄISCHE PATENTANMELDUNG

(21) Anmeldenummer: 89112061.0

(51) Int. Cl.4: C07D 473/38 , A61K 31/52

(22) Anmeldetag: 01.07.89

Patentansprüche für folgende Vertragsstaaten: ES + GR

(30) Priorität: 09.07.88 DE 3823345

(43) Veröffentlichungstag der Anmeldung: 17.01.90 Patentblatt 90/03

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Klosa, Josef, Dr.
Jänickestrasse 13
D-1000 Berlin 37(DE)
Erfinder: Kröger, Hans, Prof. Dr. Dr.
Am Schweizerhof 2b
D-1000 Berlin 37(DE)
Erfinder: Meichsner, Christoph, Dr.
Bienerstrasse 30
D-6238 Hofheim am Taunus(DE)
Erfinder: Winkler, Irvin, Dr.
In den Eichen 40
D-6237 Liederbach(DE)
Erfinder: Helsberg, Matthias, Dr.
Münsterer Strasse 14
D-6233 Kelkheim (Taunus)(DE)
Erfinder: Schrinner, Elmar, Dr.
Hedwigstrasse 2
D-6200 Wiesbaden(DE)

## (54) 6-Merkaptopurin-Derivate, ihre Herstellung und ihre Verwendung zur Bekämpfung von Retrovirusinfektionen.

(57) 6-Merkaptopurin-Derivate der Formel

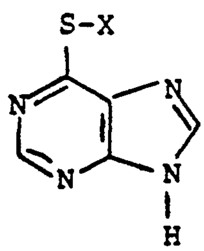

in der X die angegebenen Bedeutungen hat, eignen sich zur Bekämpfung von Retrovirusinfektionen und zur Bekämpfung von durch Retrovirusinfektion hervorgerufenen Krankheiten von Säugetieren oder dem Menschen.

EP 0 350 742 A1

# 6-Merkaptopurin-Derivate, ihre Herstellung und ihre Verwendung zur Bekämpfung von Retrovirusinfektionen

Die vorliegende Erfindung betrifft 6-Merkaptopurin-Derivate, ihre Herstellung und ihre Verwendung zur Bekämpfung von Retrovirusinfektionen.

6-Merkaptopurin ist eine Verbindung der Formel

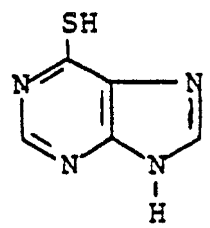

Es ist bereits bekannt, daß Derivatisierungen dieser Verbindung an der Merkaptofunktion zu pharmakologisch aktiven Verbindungen führen. So besitzt z.B. das Azathioprin, eine Verbindung der Formel (vgl. US-PS 3 056 785)

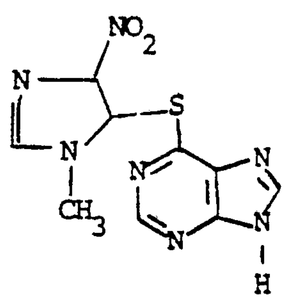

eine immunsuppressive und entzündungshemmende Wirkung.

Weiterhin wurde bereits vorgeschlagen, die lymphoblastische Leukämie im Kindesalter mit 6-Methylmercaptopurin (vgl. Lennard et al. in The Lancet, October 3, 1987) zu behandeln und 6-Dialkylaminoalkylpurine als Arzneimittel mit antithrombotischer Wirkung einzusetzen (vgl. US-PS 4 189 579).

Aufgabe der vorliegenden Erfindung ist es, antiviral wirksame Verbindungen - insbesondere gegen HIV (Human Immune Deficiency Virus) wirksame - bereitzustellen, da es bisher im allgemeinen keine befriedigende Behandlungsmöglichkeit von Viruserkrankungen, insbesondere gegen Infektionen mit dem HIV gibt. Die unzulängliche Wirkung und teilweise gravierenden Nebenwirkungen von bereits einschlägig eingesetzten Mitteln wie z.B. AZT oder Suramin sind hinlänglich bekannt.

Es wurde nun überraschenderweise gefunden, daß bestimmte 6-Merkaptopurin-Derivate eine ausgezeichnete Wirkung gegen Retrovirusinfektionen von Säugetieren und dem Menschen haben und sich zur Bekämpfung von durch Retrovirusinfektionen hervorgerufenen Krankheiten bei Säugetieren und dem Menschen eignen.

Erfindungsgegenstand sind demzufolge Purinderivate der Formel I

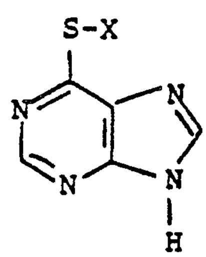

(I)

in der X eine $C_1$-$C_6$-Alkylgruppe ist, die eine oder mehrere Doppelbindungen enthalten kann und substituiert sein kann mit bis zu 3 Phenylgruppen, die ihrerseits mit bis zu 3 Halogenatomen oder Nitrogruppen substituiert sein können, oder X eine $C_1$-$C_6$-Alkylgruppe ist, die substituiert ist mit einem gesättigten oder bis zu dreifach ungesättigten stickstoff- und/oder sauerstoffhaltigen Heterocyclus mit bis zu 6 C-Atomen, bis zu 3 Stickstoffatomen und bis zu 2 Sauerstoffatomen, der seinerseits mit bis zu 3 Oxogruppen und/oder bis zu 3 $C_1$-$C_3$-Alkylgruppen substituiert sein kann, oder X eine $C_1$-$C_6$-Alkylgruppe ist, die substituiert ist mit einer oder mehreren funktionellen Gruppen ausgewählt aus der Gruppe -C≡N, -OH, $SO_3$-Alkali, -$COOR^1$ und -$NR^1R^2$, worin $R^1$ und $R^2$ unabhängig voneinander Wasserstoff oder verzweigte oder unverzweigte $C_1$-$C_6$-Alkylgruppen sind oder X eine Cycloalkylgruppe mit 4 - 6 Ringatomen ist, in der ein Ringatom durch O, NH oder $NH_2$ ersetzt sein kann und die substituiert sein kann mit einer Oxogruppe und/oder einer Aminogruppe sowie deren physiologisch vertragliche Salze, mit Ausnahme der Verbindungen, in denen X eine Methylgruppe eine Dialkylaminoalkoholgruppe oder eine Gruppe der Formel II ist

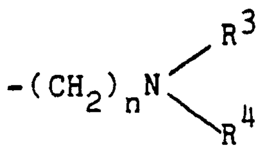

II

in der n = 1 bis 3 ist und $R^3$ eine Alkylgruppe mit 1 bis 4 C-Atomen ist und $R^4$ Wasserstoff oder eine Alkylgruppe mit 1 oder 2 C-Atomen ist.

Weiterhin gehört zum Erfindungsgegenstand die Verwendung von Verbindungen der Formel Ia

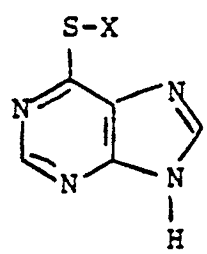

Ia

wobei X eine $C_1$-$C_6$-Alkylgruppe ist, die eine oder mehrere Doppelbindungen enthalten kann und substituiert sein kann mit bis zu 3 Phenylgruppen, die ihrerseits mit bis zu 3 Halogenatomen oder Nitrogruppen substituiert sein können oder X eine $C_1$-$C_6$-Alkylgruppe ist, die substituiert ist mit einem gesättigten oder bis zu dreifach ungesättigten stickstoff- und/oder sauerstoffhaltigen Heterocyclus mit bis zu 6 C-Atomen, bis zu 3 Stickstoffatomen und bis zu 2 Sauerstoffatomen, der seinerseits mit bis zu 3 Oxogruppen und/oder bis zu 3 $C_1$-$C_3$-Alkylgruppen substituiert sein kann oder X eine $C_1$-$C_6$-Alkylgruppe ist, die substituiert ist mit einer oder mehreren funktionellen Gruppen, ausgewählt aus der Gruppe -C≡N, -OH, -$SO_3$-Alkali, -$COOR^1$ und -$NR^1R^2$, worin $R^1$ und $R^2$ unabhängig voneinander Wasserstoff oder verzweigte oder unverzweigte $C_1$-$C_6$-Alkylgruppen sind oder X eine Cycloalkylgruppe mit 4 - 6 Ringatomen ist, in der ein Ringatom durch O, NH oder $NH_2$ ersetzt sein kann und die substituiert sein kann mit einer Oxogruppe und/oder einer Aminogruppe sowie deren physiologisch vertraglichen Salzen zur Bekämpfung von Retrovirusinfektionen oder durch Retrovirusinfektion hervorgerufenen Krankheiten bei Säugetieren oder dem Menschen.

Bevorzugt ist die Verwendung von Verbindungen, in denen X eine $C_1$-$C_3$-Alkyl- oder Alkenylgruppe ist, die mit einer Biphenyl-, Urazil-, Morpholino- oder Pyridyl-Gruppe, mit bis zu 3 Hydroxylgruppen, mit einer $C_1$-$C_3$-Alkylestergruppe oder einer $C_1$-$C_3$-Dialkylaminogruppe substituiert sein kann.

Ganz besonders bevorzugt ist die Verwendung von Verbindungen, in denen X eine 2-Propenyl-, eine 3-Pyridylmethyl- oder 4-Pyridylmethyl- oder eine 3-Diisopropylaminopropyl-Gruppe ist.

Gegenstand der Erfindung sind weiterhin Arzneimittel, die einen Gehalt an Verbindungen der Formel I oder Ia bzw. deren physiologisch verträglichen Salzen aufweisen. Die genannten Arzneimittel eignen sich insbesondere zur Bekämpfung von Retrovirusinfektionen und Erkrankungen, die durch Retrovirusinfektion, z.B. HIV bei Säugetieren oder dem Menschen hervorgerufen .worden sind. Die erfindungsgemäßen Arzneimittel können in geeigneter Form zur peroralen, rektalen oder parenteralen Verabreichung, beispielsweise in Form von Hartkapseln, Tabletten, Granulat, Weichkapseln oder wässrigen Lösungen, Sirups oder trinkbaren Suspensionen vorliegen. Die Zubereitungen enthalten den Wirkstoff und vorzugsweise pharmazeutisch unbedenkliche Zusatzstoffe, wie Verdünnungsmittel und/oder Trägermaterialien. Hierunter sind physiologisch unbedenkliche Substanzen zu verstehen, die nach dem Vermischen mit dem Wirkstoff diesen in eine für die Verabreichung geeignete Form bringen.

Als häufig verwendete Trägerstoffe bzw. Verdünnungsmittel seien z.B. verschiedene Zucker oder Stärkearten, Cellulosederivate, Magnesiumcarbonat, Magnesiumstearat, Talk, Gelatine, colloidales Siliziumdioxid, tierische und pflanzliche Öle, Polyethylenglykole, Wasser oder andere geeignete Lösungsmittel sowie wasserhaltige Puffermittel, die durch Zusatz von Glukose oder Salzen isotonisch gemacht werden können, genannt. Außerdem können gegebenenfalls oberflächenaktive, Farb- und Geschmacksstoffe, Stabilisatoren sowie Konservierungsmittel als weitere Zusatzstoffe in den erfindungsgemäßen Arzneimittelzubereitungen Verwendung finden. Der Wirkstoffanteil beträgt 10 bis 100 Gew.-%, vorzugsweise 25 bis 75 Gew.-%.

Die tägliche Dosierung der wirksamen Verbindungen kann in einem weiten Bereich variiert werden. Zu bevorzugen sind 0,1 bis 10, insbesondere 0,2 bis 8 mg Wirkstoff/kg Körpergewicht.

Die zu verabreichende Menge kann auf mehrere Dosierungseinheiten verteilt werden. Gegebenenfalls können die Dosierungseinheiten für die orale Verabreichung mikroverkapselt werden, um die Wirkstoffabgabe zu verzögern oder über einen längeren Zeitraum auszudehnen. Die Mikroverkapselung erfolgt nach allgemein bekannten Methoden, z.B. durch Überziehen oder Einbetten in geeignete Polymere, Wachse oder dergl..

Die parenterale Verabreichung kann unter Verwendung flüssiger Dosierungsformen, wie steriler Lösungen und Suspensionen erfolgen, die für die intramuskuläre oder subkutane Injektion bestimmt sind. Derartige Dosierungsformen werden durch Lösen bzw. Suspendieren einer abgemessenen Wirkstoffmenge in einem geeigneten physiologisch unbedenklichen Verdünnungsmittel, wie beispielsweise einem wäßrigen oder öligen Medium und Sterilisierung der Lösung bzw. der Suspension, gegebenenfalls unter Mitverwendung von geeigneten Stabilisatoren, Emulgatoren und/oder Konservierungsmittel, hergestellt. Die pharmazeutischen Präparate werden nach allgemein üblichen Verfahren hergestellt.

Weiterhin richtet sich die vorliegende Erfindung auf ein Verfahren zur Herstellung von Verbindungen der Formel I, das dadurch gekennzeichnet ist, daß eine Verbindung der Formel III

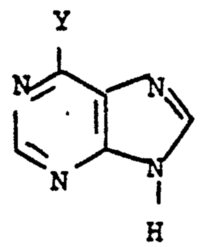

(III)

mit einer Verbindung der Formel H-S-X, wobei Y Halogen ist und X die zu Formel I genannten Bedeutungen hat, umgesetzt wird. Die Umsetzung erfolgt vorzugsweise mit einer Verbindung der Formel III, in der das Halogenatom Brom oder Chlor ist. Ein geeignetes Reaktionsmedium ist z.B. eine alkoholische Lösung unter Zusatz einer Base, bevorzugt Trialkylamin in Ethanol. Die Reaktionspartner werden vorzugsweise in etwa äquimolaren Mengen eingesetzt. Die Umsetzung wird normalerweise bei Raumtemperatur durchgeführt, es kann aber auch zweckmäßig sein, die Reaktionsmischung zu erhitzen.

Ein weiteres Verfahren zur Herstellung erfindungsgemäß verwendbarer Verbindungen ist dadurch gekennzeichnet, daß eine Verbindung der Formel IV

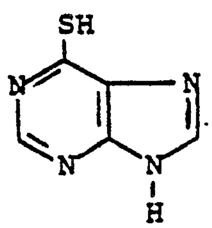

(IV)

mit einer Verbindung der Formel Y-X, in der Y Halogen, vorzugsweise Chlor oder Brom ist und X die zu Formel Ia genannten Bedeutungen hat, umgesetzt wird. Die Umsetzung wird vorzugsweise - unter überraschend milden Bedingungen - in wäßrig/alkoholischer Lösung unter Basenzusatz durchgeführt. Bevorzugt

ist eine wäßrige Lösung mit einem Ethanolanteil von ca. 40 bis 60 % unter Zusatz von Natriumhydroxid. Die Reaktion erfolgt zweckmäßigerweise bei Raumtemperatur oder unter leichtem Erhitzen. Nach Beendigung der Reaktion kann das Produkt durch übliche Trennverfahren, z.B. Filtration, aus der Reaktionsmischung isoliert werden und gegebenenfalls durch übliche Methoden, z.B. Umkristallisation, weiter gereinigt werden.

Die nicht halogenierten Ausgangsprodukte für die beschriebenen Reaktionen sind handelsübliche Substanzen. Die halogenierten Ausgangssubstanzen sind größtenteils ebenfalls handelsüblich oder können auf einfache Weise synthetisiert werden (vgl. z.B. Organikum, 15. Aufl. VEB Deutscher Verlag der Wissenschaften, Berlin 1977, S. 200 ff.).

Durch die nachfolgenden Ausführungsbeispiele und Wirksamkeitstests soll die Erfindung näher erläutert werden.

**Beispiel 1**

**Herstellung von 6-(4-pyridylmethyl)-mercaptopurin (Präparat 1)**

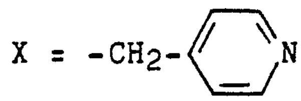

5,6g KOH werden in einem Gemisch von 25 ml Wasser und 25 ml Ethanol gelöst. Unter Rühren werden 8,5 g 6-Mercaptopurin eingetragen. Anschließend werden bei Zimmertemperatur 8,5 g 4-Picolylmethyl-chlorid-hydrochlorid in 20 ml Wasser unter Rühren zugetropft. Das Reaktionsgut färbt sich tief weinrot. Unter leichtem Temperaturanstieg sinkt der pH-Wert auf 7,5 bis 8,5. Nach beendeter 4-Picolylmethyl-chloridhydrochloridzugabe wird weiter 15 Minuten gerührt, über Nacht stehen gelassen und abgesaugt. Es werden 12,5 g Produkt erhalten. Die Base ist unlöslich in Wasser, aber gut löslich in allen säurehaltigen Flüssigkeiten.

Fp.: 270/271 °C.

Das Hydrochlorid ist erhältlich, indem man die Base in wenig alkoholischer Salzsäure löst, mit Aceton oder Ether verdünnt und den erhaltenen Niederschlag absaugt.

Fp.: 225/227 °C

**Beispiel 2**

**Herstellung von 6-(3-Pyridylmethyl)-mercaptopurin (Präparat 2)**

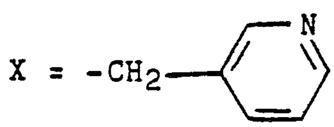

Die Darstellung dieser Verbindung verläuft wie zu Beispiel 1 beschrieben, unter Verwendung von 3-Picolylmethyl-chlorid hydrochlorid. Die Ausbeute beträgt 12 g. Das Produkt ist ein graues Pulver.

Fp.: 200/202 °C. Hydrochlorid Fp.: 218/220 °C.

**Beispiel 3**

**6-(2-propenyl)-mercaptopurin (Präparat 3)**

X = $-CH_2-CH=CH_2$

3 g KOH werden in 20 ml Wasser und 20 ml Alkohol gelöst. Unter Rühren werden 8,5 g 6-Mercaptopurin zugegeben und anschließend werden ebenfalls unter Rühren 7 g 3-Brompropen-1 zugetropft. Das Umsetzungprodukt fällt als dicker Kristallbrei aus, der abgesaugt und mit Wasser gewaschen wird. Die Umkristallisation erfolgt aus Wasser und Alkohol im Verhältnis 1:1 oder durch Losen in Alkohol und Zellen mit Wasser. Das Produkt ist leicht löslich in Alkohol, heißem Wasser und Polyethylenglykol. Schwer löslich ist es in kaltem Wasser.
Ausbeute: 9,5 g Fp.: 171/173° C.

**Beispiel 4**

**Herstellung von 6-(3-Dimethylaminopropyl)-mercaptopurin**

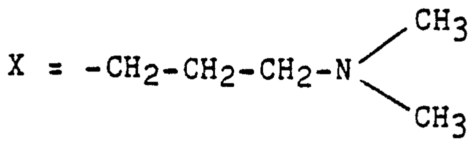

5,6 g KOH werden in 40 ml Wasser gelöst und 8,5 g 6-Mercaptopurin werden zugegeben. In diese Lösung läßt man unter Rühren eine Lösung von 8 g Dimethylaminopropyl-chloridhydrochlorid in 20 ml Wasser eintropfen. Nach Beendigung der Zugabe wird 30 Minuten unter Erhitzen auf ca. 100° C gerührt. Nach dem Erkalten wird NaOH bis zur alkalischen Reaktion zugegeben. Die freie Base hat einen Fp.: von 86/88° C.
Das Hydrochlorid ist erhältlich durch Lösen in alkoholischer Salzsäure und Verdünnen mit Aceton oder Ether.
Das Hydrochlorid ist ein farbloses Pulver.
Fp.: 188/290° C.

**Beispiel 5**

**Herstellung von 6-(3-Diisopropylaminopropyl)-mercaptopurin (Präparat 4)**

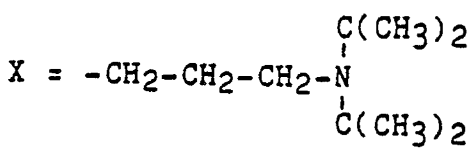

Wie zu Beispiel 4 beschrieben, erhält man unter Verwendung von 1-Chlor-3-diisopropylaminopropan-hydrochlorid die genannte Verbindung. Die freie Base ist ein farbloses Pulver.
Fp.: 96/98° C. Hydrochlorid Fp.: 194/196° C.

Nachfolgend sind weitere Beispiele in tabellarischer Form mit der Angabe von Schmelzpunkten aufgeführt.

| Bsp. | Verbindung | X | Fp. (°C) |
|---|---|---|---|
| 6 | 6-(2-Hydroxyethyl)-mercaptopurin | $-CH_2-CH_2-OH$ | 178/180 |
| 7 | 6-(3-Hydroxypropyl)-mercaptopurin | $-CH_2-CH_2-CH_2-OH$ | 156/158 |
| 8 | 6-(2,3-Dihydroxypropyl)-mercaptopurin | $-CH_2-CH_2-CH-CH_2-OH$<br>OH | - |
| 9 | 6-(p-Nitrophenacyl)-mercaptopurin | 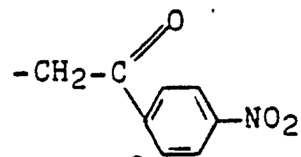 | 150/152 |
| 10 | 6-(3-(2-Oxo-tetrahydro-furyl))-mercaptopurin | 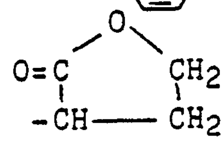 | 190/192 |
| 11 | 6-(2-Oxo-3-ethoxy-carbonylpropyl)-mercaptopurin | 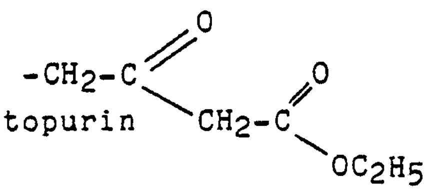 | 55/ 58 |
| 12 | 6-(2-Oxoethyl)-mercaptopurin | 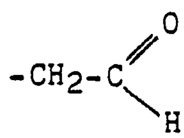 | 188/190 |
| 13 | 6-(Ethoxycarbonylmethyl)-mercaptopurin | 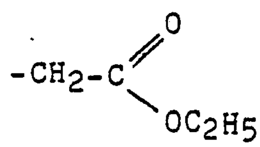 | 108/110 |
| 14 | 6-(2-Diethylaminoethyl)-mercaptopurin | 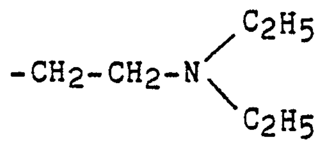 | 220/222 |
| 15 | 6-(2-N-Piperidinoethyl)-mercaptopurin (Tartrat) | 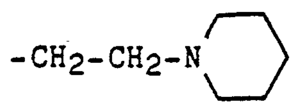 | 272/274 |
| 16 | 6-(2-N-Morpholinoethyl)-mercaptopurin | 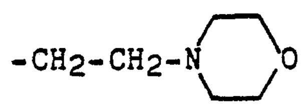 | - |

Blockierung der Reverse Transkriptase-(RT)-Aktivität untersucht wird, erscheint die Wahl eines solchen Ersatzmodelles gerechtfertigt. Im vorliegenden Fall wurde die Infektion der Maus mit dem Friend-Leukämie-Virus gewählt. Dazu wurden normale Labormäuse (NMRI = Naval Medical Research Institute) durch intravenöse Injektion mit Friend-Virus enthaltendem Mäuseserum infiziert. Bei den unbehandelten Kontrolltieren entwickelte sich als Symptom der Infektion innerhalb von 2 Wochen eine deutliche Vergrößerung von Milz und Leber. Die Behandlung erfolgte über 10 Tage, beginnend 48 Stunden nach der Infektion. Am 14. Versuchstag wurden die Tiere durch Luxation der Halswirbel getötet und geöffnet. Die Milz wurde entnommen und gewogen. Als Meßparameter der therapeutischen Wirksamkeit wurde das Milzgewicht der behandelten Tiere mit dem der unbehandelten Infektionskontrolle in Relation gesetzt.

Während bei uninfizierten ausgewachsenen Labormäusen (20 -24 g Körpergewicht) die Milz etwa 1 % des Körpergewichtes oder weniger wog, erreichte bei infizierten Tieren die Milz am Ende des Versuchs etwa 10 % des Körpergewichts.

Tabelle I zeigt die Ergebnisse der Wirksamkeitstests bei der Friend-Leukämie-Virusinfektion in der Maus bei intraperitonealer Verabreichung der erfindungsgemäßen Verbindungen. Als Kontrolle diente eine Gruppe von Mäusen, die unbehandelt blieben.

Zum Vergleich wurde eine weitere Gruppe von Mäusen mit Suramin behandelt. Tabelle I verdeutlicht, daß die erfindungsgemäßen Verbindungen besser wirken als Suramin, bei vergleichbarer oder geringerer Beeinträchtigung des Hämaglobingehaltes des Blutes. Bei Verabreichung der erfindungsgemäßen Verbindungen überlebten alle Mäuse die Behandlung, wohingegen bei der Verabreichung von Suramin 3 von 10 Mäusen starben. Weiterhin war der Behandlungserfolg, erkennbar an der Verringerung der Milzschwellung, bei Verabreichung aller getesteten Verbindungen größer als bei der Verabreichung von Suramin.

Ein ähnliches Ergebnis zeigte sich bei dem gleichen Wirksamkeitstest bei oraler Verabreichung der Wirkstoffe. Hier wurde neben Suramin noch AZT (Azidothymidin) als Vergleichssubstanz herangezogen. AZT hat zumindest zu Behandlungsbeginn eine hervorragende Wirksamkeit bei der Bekämpfung der durch eine HIV-Infektion hervorgerufenen Immunschwäche, verursacht aber erhebliche Nebenwirkungen, die eine Dauertherapie fragwürdig erscheinen lassen (vgl. Münch med. Wschr. 129 (1987) Nr. 42, S. 32). Tabelle II zeigt, daß die erfindungsgemäßen Substanzen bei oraler Verabreichung wiederum wirksamer waren als Suramin und eine durchaus vergleichbare Wirksamkeit wie das AZT aufwiesen.

Tabelle I

| **Wirkung gegen FLV in der Maus, Applikation i.p.** | | | | | |
|---|---|---|---|---|---|
| Präparat | Dosis mg/Maus | rel. Milzgewicht % Körpergewicht | n* | Signifikanz P-Wert | Hg g/ % |
| Kontrolle | 0 | 7.63 ± 2.40 | 10 | 1.0000 | 13.9 |
| Suramin | 10 x 1.0 i.p. | 4.54 ± 1.86 | 7 | 0.0060 | 11.7 |
| 1 | 10 x 0.1 i.p. | 5.23 ± 2.69 | 10 | 0.0235 | |
| | 10 x 1.0 i.p. | 2.08 ± 0.44 | 10 | 0.0000 | 13.2 |
| 2 | 10 x 0.1 i.p. | 5.46 ± 3.32 | 10 | 0.0534 | |
| | 10 x 1.0 i.p. | 1.84 ± 0.82 | 10 | 0.0000 | 11.6 |
| 3 | 10 x 0.1 i.p. | 5.86 ± 1.84 | 10 | 0.0385 | |
| | 10 x 1.0 i.p. | 1.37 ± 0.38 | 10 | 0.0000 | 13.9 |
| 4 | 10 x 0.1 i.p. | 6.11 ± 1.71 | 10 | 0.0583 | |
| | 10 x 1.0 i.p. | 3.44 ± 2.33 | 10 | 0.0006 | 11.3 |
| Negativkontrolle- stets ≤1 % | | | - | - | |

*Anzahl überlebende Mäuse

Tabelle II

Wirkung gegen FLV in der Maus, Applikation oral über Trinkwasser

| Präparat | Dosis mg/Maus | rel. Milzgewicht % Körpergewicht | n* | Signifikanz P-Wert | Hg g/% |
|---|---|---|---|---|---|
| Kontrolle | 0 | 7.88 ± 2.95 | 10 | 1.0000 | 10.8 |
| (Suramin | 10 x 1.00 i.p. | 4.50 ± 2.22 | 9 | 0.0061 | 10.0) |
| AZT | 0.05 mg/ml<br>10 x 0.18 mg/Maus | 2.80 ± 1.66 | 10 | 0.0001 | 14.1 |
| AZT | 0.10 mg/ml<br>10 x 0.34 mg/Maus | 2.67 ± 2.11 | 10 | 0.0002 | 12.7 |
| | 0.05 mg/ml<br>10 x 0.17 mg/Maus | 7.01 ± 2.99 | 10 | 0.2634 | |
| 1 | 0.10 mg/ml<br>10 x 0.36 mg/Maus | 4.80 ± 2.74 | 10 | 0.0124 | |
| | 0.50 mg/ml<br>10 x 1.69 mg/Maus | 1.66 ± 1.25 | 10 | 0.0000 | 11.3 |
| | 0.05 mg/ml<br>10 x 0.17 mg/Maus | 5.76 ± 2.72 | 10 | 0.0541 | |
| 2 | 0.10 mg/ml<br>10 x 0.36 mg/Maus | 1.46 ± 1.00 | 10 | 0.0000 | |
| | 0.50 mg/ml<br>10 x 1.67 mg/Maus | 0.90 ±1.12 | 10 | 0.0000 | 10.4 |
| | 0.05 mg/ml<br>10 x 0.17 mg/Maus | 4.52 ± 2.28 | 10 | 0.0051 | |
| 3 | 0.10 mg/ml<br>10 x 0.33 mg/Maus | 1.51 ± 1.26 | 10 | 0.0000 | |

Tabelle II (Forts.)

**Wirkung gegen FLV in der Maus, Applikation oral über Trinkwasser**

| Präparat | Dosis mg/Maus | rel. Milzgewicht % Körpergwicht | n* | Signifikanz P-Wert | Hg g/% |
|---|---|---|---|---|---|
| | 0.05 mg/ml<br>10 x 0.17 mg/Maus | 8.78 ± 2.30 | 10 | 0.2297 | |
| 4 | 0.10 mg/ml<br>10 x 0.33 mg/Maus | 8.23 ± 4.43 | 10 | 0.4152 | |
| | 0.50 mg/ml<br>10 x 1.77 mg/Maus | 2.72 ± 1.23 | 10 | 0.0000 | 12.7 |
| Negativkontrolle | - | stets <1 % | - | - | |

* Anzahl überlebende Mäuse

Präp. 1 = 6-(4-Pyridylmethyl)-mercaptopurin (Bsp. 1)
Präp. 2 = 6-(3-Pyridylmethyl)-mercaptopurin (Bsp. 2)
Präp. 3 = 6-(2-Propenyl)-mercaptopurin (Bsp. 3)
Präp. 4 = 6-(3-Diisopropylaminopropyl)-mercaptopurin (Bsp. 5)

## Ansprüche

1. Purinderivate der Formel I

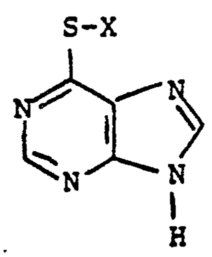

(I)

in der X eine $C_1$-$C_6$-Alkylgruppe ist, die eine oder mehrere Doppelbindungen enthalten kann und substituiert sein kann mit bis zu 3 Phenylgruppen, die ihrerseits mit bis zu 3 Halogenatomen oder Nitrogruppen

substituiert sein können oder X eine $C_1$-$C_6$-Alkylgruppe ist, die substituiert ist mit einem gesättigten oder bis zu dreifach ungesättigten stickstoff- und/oder sauerstoffhaltigen Heterocyclus mit bis zu 6 C-Atomen, bis zu 3 Stickstoffatomen und bis zu 2 Sauerstoffatomen, der seinerseits mit bis zu 3 Oxogruppen und/oder bis zu 3 $C_1$-$C_3$-Alkylgruppen substituiert sein kann oder X eine $C_1$-$C_6$-Alkylgruppe ist, die substituiert ist mit einer oder mehreren funktionellen Gruppen ausgewählt aus der Gruppe -C≡N, -OH, -$SO_3$-Alkali, -$COOR^1$ und -$NR^1R^2$, worin $R^1$ und $R^2$ unabhängig voneinander Wasserstoff oder verzweigte oder unverzweigte $C_1$-$C_6$-Alkylgruppen sind, oder X eine Cycloalkylgruppe mit 4 - 6 Ringatomen ist, in der ein Ringatom durch O, NH oder $NH_2$ ersetzt sein kann und die substituiert sein kann mit einer Oxogruppe und/oder einer Aminogruppe sowie deren physiologisch vertragliche Salze, mit Ausnahme der Verbindungen, in denen X eine Methylgruppe, eine Dialkylaminoalkoholgruppe oder eine Gruppe der Formel II ist

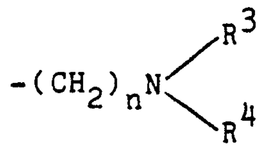

II,

in der n = 1 bis 3 ist und $R^3$ eine Alkylgruppe mit 1 bis 4 C-Atomen ist und $R^4$ Wasserstoff oder eine Alkylgruppe mit 1 oder 2 C-Atomen ist.

2. Verwendung von Verbindungen der Formel Ia

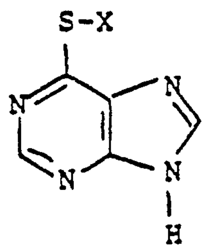

(Ia)

in der X eine $C_1$-$C_6$-Alkylgruppe ist, die eine oder mehrere Doppelbindungen enthalten kann und substituiert sein kann mit bis zu 3 Phenylgruppen, die ihrerseits mit bis zu 3 Halogenatomen oder Nitrogruppen substituiert sein können oder X eine $C_1$-$C_6$-Alkylgruppe ist, die substituiert sein kann mit einem gesättigen oder bis zu dreifach ungesättigten stickstoff- und/oder sauerstoffhaltigen Heterocyclus mit bis zu 6 C-Atomen, bis zu 3 Stickstoffatomen und bis zu 2 Sauerstoffatomen, der seinerseits mit bis zu 3 Oxogruppen und/oder bis zu 3 $C_1$-$C_3$-Alkylgruppen substituiert sein kann oder X eine $C_1$-$C_6$-Alkylgruppe ist, die substituiert ist mit einer oder mehreren funktionellen Gruppen, ausgewählt aus der Gruppe -C≡N, -OH, -$SO_3$-Alkali, -$COOR^1$ und -$NR^1R^2$, worin $R^1$ und $R^2$ unabhängig voneinander Wasserstoff oder verzweigte oder unverzweigte $C_1$-$C_6$-Alkylgruppen sind, oder X eine Cycloalkylgruppe mit 4 - 6 Ringatomen ist, in der ein Ringatom durch O, NH oder $NH_2$ ersetzt sein kann und die substituiert sein kann mit einer Oxogruppe und/oder einer Aminogruppe sowie deren physiologisch vertraglichen Salzen zur Bekämpfung von Retrovirusinfektionen oder von durch Retrovirusinfektion hervorgerufenen Krankheiten bei Säugetieren oder dem Menschen.

3. Verwendung von Verbindungen gemäß Anspruch 2, dadurch gekennzeichnet, daß X eine $C_1$-$C_3$-Alkyl- oder Alkenylgruppe ist, die mit einer Diphenyl-, Urazil-, Morpholino-, oder Pyridylgruppe, mit bis zu 3 Hydroxylgruppen, mit einer $C_1$-$C_3$-Alkylestergruppe oder einer $C_1$-$C_3$-Dialkylaminogruppe substituiert sein kann.

4. Verwendung von Verbindungen gemäß Anspruch 2 oder 3, dadurch gekennzeichnet, daß X eine 2-Propenyl-, eine 3-Pyridylmethyl- oder 4-Pyridylmethyl- oder eine 3-Diisopropylaminopropyl-Gruppe ist.

5. Arzneimittel, gekennzeichnet durch einen Gehalt an einer oder mehreren Verbindungen gemäß Anspruch 1.

6. Arzneimittel gekennzeichnet durch einen Gehalt and einer oder mehreren Verbindungen gemäß Anspruch 2, zur Bekämpfung von Retrovirusinfektionen von Säugetieren oder dem Menschen.

7. Verwendung von Verbindungen der Formel I gemäß Anspruch 1 zur Bekämpfung von Erkrankungen, die durch Retrovirusinfektionen von Säugetieren oder dem Menschen hervorgerufen worden sind.

8. Verwendung von Verbindungen der Formel Ib

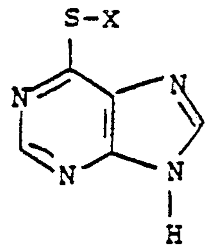

(Ib)

in der X ein Substituent der Formel II gemäß Anspruch 1 ist oder deren physiologisch verträglichen Salzen zur Bekämpfung von Retrovirusinfektionen oder von Erkrankungen, die durch Retrovirusinfektion von Säugetieren oder dem Menschen hervorgerufen worden sind.

9. Verfahren zur Herstellung von Verbindungen der Formel I gemäß Anspruch 1, dadurch gekennzeichnet, daß eine Verbindung der Formel III

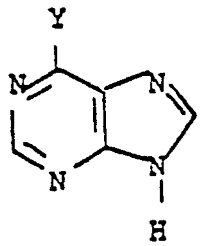

(III)

mit einer Verbindung der Formel H-S-X, wobei Y Halogen ist und X die in Anspruch 1 genannten Bedeutungen hat, umgesetzt wird.

10. Verfahren zur Herstellung von Verbindungen der Formel I gemäß Anspruch 1, dadurch gekennzeichnet, daß eine Verbindung der Formel IV

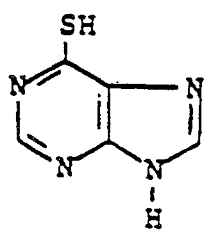

IV

mit einer Verbindung der Formel Y-X, in der Y ein Halogen ist und X die in Anspruch 1 genannten Bedeutungen hat, in wässriger alkoholischer Alkalihydroxidlösung umgesetzt wird.

Patentansprüche für folgende Vertragsstaaten: ES, GR

1. Verfahren zur Herstellung von Purinderivaten der Formel I

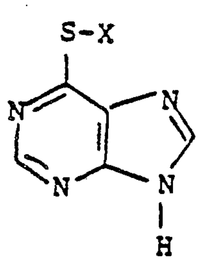

(I)

in der X eine $C_1$-$C_6$-Alkylgruppe ist, die eine oder mehrere Doppelbindungen enthalten kann und substituiert sein kann mit bis zu 3 Phenylgruppen, die ihrerseits mit bis zu 3 Halogenatomen oder Nitrogruppen substituiert sein können oder X eine $C_1$-$C_6$-Alkylgruppe ist, die substituiert ist mit einem gesättigten oder

bis zu dreifach ungesättigten stickstoff- und/oder sauerstoffhaltigen Heterocyclus mit bis zu 6 C-Atomen, bis zu 3 Stickstoffatomen und bis zu 2 Sauerstoffatomen, der seinerseits mit bis zu 3 Oxogruppen und/oder bis zu 3 $C_1$-$C_3$-Alkylgruppen substituiert sein kann oder X eine $C_1$-$C_6$-Alkylgruppe ist, die substituiert ist mit einer oder mehreren funktionellen Gruppen ausgewählt aus der Gruppe -C≡N, -OH, $-SO_3$ -Alkali, $-COOR^1$ und $NR^1R^2$ worin $R^1$ und $R^2$ unabhängig voneinander Wasserstoff oder verzweigte oder unverzweigte $C_1$-$C_6$-Alkylgruppen sind, oder X eine Cycloalkylgruppe mit 4 - 6 Ringatomen ist, in der ein Ringatom durch O, NH oder $NH_2$ ersetzt sein kann und die substituiert sein kann mit einer Oxogruppe und/oder einer Aminogruppe sowie deren physiologisch verträgliche Salze, mit Ausnahme der Verbindungen, in denen X eine Methylgruppe, eine Dialkylaminoalkoholgruppe oder eine Gruppe der Formel II ist

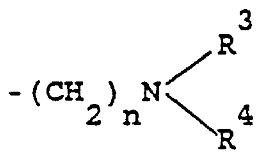

II,

in der n = 1 bis 3 ist und $R^3$ eine Alkylgruppe mit 1 bis 4 C-Atomen ist und $R^4$ Wasserstoff oder eine Alkylgruppe mit 1 oder 2 C-Atomen ist, dadurch gekennzeichnet, daß eine Verbindung der Formel III

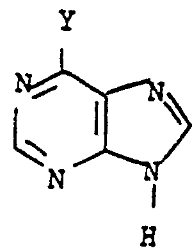

(III)

mit einer Verbindung der Formel H-S-X, wobei Y Halogen ist und X die genannten Bedeutungen hat, umgesetzt wird, oder
daß eine Verbindung der Formel IV

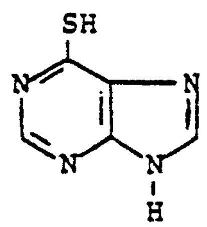

IV

mit einer Verbindung der Formel Y-X, in der Y ein Halogen ist und X die genannten Bedeutungen hat, in wässriger alkoholischer Alkalihydroxidlösung umgesetzt wird.

2. Verwendung von Verbindungen der Formel Ia

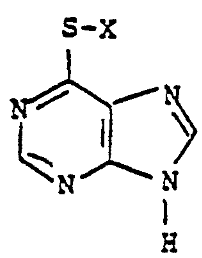

(Ia) ,

in der X eine $C_1$-$C_6$-Alkylgruppe ist, die eine oder mehrere Doppelbindungen enthalten kann und substituiert sein kann mit bis zu 3 Phenylgruppen, die ihrerseits mit bis zu 3 Halogenatomen oder Nitrogruppen

substituiert sein können oder X eine $C_1$-$C_6$-Alkylgruppe ist, die substituiert sein kann mit einem gesättigen oder bis zu dreifach ungesättigten stickstoff- und/oder sauerstoffhaltigen Heterocyclus mit bis zu 6 C-Atomen, bis zu 3 Stickstoffatomen und bis zu 2 Sauerstoffatomen, der seinerseits mit bis zu 3 Oxogruppen und/oder bis zu 3 $C_1$-$C_3$-Alkylgruppen substituiert sein kann oder X eine $C_1$-$C_6$-Alkylgruppe ist, die substituiert ist mit einer oder mehreren funktionellen Gruppen, ausgewählt aus der Gruppe -C≡N, -OH, -$SO_3$ -Alkali, -$COOR^1$ und $NR^1R^2$, worin $R^1$ und $R^2$ unabhängig voneinander Wasserstoff oder verzweigte oder unverzweigte $C_1$-$C_6$-Alkylgruppen sind, oder X eine Cycloalkylgruppe mit 4 - 6 Ringatomen ist, in der ein Ringatom durch O, NH oder $NH_2$ ersetzt sein kann und die Substituiert sein kann mit einer Oxogruppe und/oder einer Aminogruppe sowie deren physiologisch Vertraglichen Salzen zur Bekämpfung von Retrovirusinfektionen oder von durch Retrovirusinfektion hervorgerufenen Krankheiten bei Säugetieren oder dem Menschen.

3. Verwendung von Verbindungen gemäß Anspruch 2, dadurch gekennzeichnet, daß X eine $C_1$-$C_3$-Alkyl- oder Alkenylgruppe ist, die mit einer Diphenyl-, Urazil-, Morpholino-, oder Pyridylgruppe, mit bis zu 3 Hydroxylgruppen, mit einer $C_1$-$C_3$-Alkylestergruppe oder einer $C_1$-$C_3$-Dialkylaminogruppe substituiert sein kann.

4. Verwendung von Verbindungen gemäß Anspruch 2 oder 3, dadurch gekennzeichnet, daß X eine 2-Propenyl-, eine 3-Pyridylmethyl oder 4-Pyridylmethyl oder eine 3-Diisopropylaminopropyl-Gruppe ist.

5. Verfahren zur Herstellung von Arzneimitteln, dadurch gekennzeichnet, daß man eine oder mehrere Verbindungen gemäß Anspruch 1 ind eine geeignete Darreichungsform bringt.

6. Verfahren zur Herstellung von Arzneimitteln zur Bekämpfung von Retrovirusinfektionen von Säugetieren oder dem Menschen, dadurch gekennzeichnet, daß man eine oder mehrere Verbindungen gemäß Anspruch 2 in eine geeignete Darreichungsform bringt.

7. Verwendung von Verbindungen der Formel I gemäß Anspruch 1 zur Bekämpfung von Erkrankungen, die durch Retrovirusinfektionen von Säugetieren oder dem Menschen hervorgerufen worden sind.

8. Verwendung von Verbindungen der Formel Ib

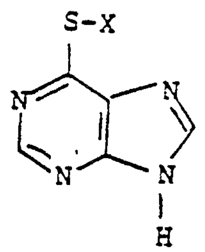

(Ib)

in der X ein Substituent der Formel II gemäß Anspruch 1 ist oder deren physiologisch verträglichen Salzen zur Bekämpfung von Retrovirusinfektionen oder von Erkrankungen, die durch Retrovirusinfektion von Säugetieren oder dem Menschen hervorgerufen worden sind.

Europäisches Patentamt

# EUROPÄISCHER RECHERCHENBERICHT

Nummer der Anmeldung

EP 89 11 2061

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,D | US-A-4 189 579 (J. E. DUNBAR et al.)<br>* Spalte 1, Zeile 10-63 *<br>--- | 1 | C 07 D 473/38<br>A 61 K 31/52 |
| A | EP-A-0 206 497 (THE WELLCOME FOUNDATION LTD.)<br>* Seite 20, Ansprüche 1-6 *<br>--- | 1,2 | |
| A | US-A-4 383 114 (R. VINCE)<br>* Spalte 12, Anspruch 1 *<br>--- | | |
| A | PHARMAZIE<br>Band 28, Heft 11/12, 1973, Seiten 795-796; G. WAGNER et al.: "Synthese und biologische Wirkung von Antigen-Mercaptopurin-Konjugation" * Seiten 795,796 *<br>--- | 1,2 | |
| A | PHARMAZIE<br>Band 27, Heft 5, 1972, Seiten 298,299; A. CERNY et al.: "Antineoplastisch wirksame Stoffe" * Seiten 298,299 *<br>----- | 1,2 | RECHERCHIERTE SACHGEBIETE (Int. Cl.5)<br>C 07 D 473/00 |

Der vorliegende Recherchenbericht wurde für alle Patentansprüche erstellt

| Recherchenort | Abschlußdatum der Recherche | Prüfer |
|---|---|---|
| BERLIN | 28-09-1989 | KYRIAKAKOU G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)